# EUROPEAN PATENT APPLICATION

(11) **EP 4 573 896 A1**
(43) Date of publication of application: **25.06.2025**
(21) Application number: 23220131.9
(22) Date of filing: 22.12.2023
(51) Int. Cl.: A01K 67/033

(54) **DEVICE AND PROCESS FOR TRANSFERRING INSECTS**

(71) Applicant: Nasekomo AD, Obshtina Stolichna, 1151 Selo Lozen (BG)
(72) Inventor: Bolard, Marc Louis Raymond, 1407 Sofia (BG); Vasilev, Kamen Nikolaev, 1000 Sofia (BG)
(74) Representative: Fidal Innovation

(57) **Abstract**

Device for transferring insects from an initial container into a final container comprising:
- a conveyor belt (11) comprising a transfer surface having a first and a second opposite side, the first side being configured for receiving insects from the initial container, the insects being received at an entry area (A) of the first side of the conveyor belt (11), the first side of the conveyor belt being further configured for transporting the insects from the entry area (A) to an exit area (B) where the insects are configured to be received in the final container, and
- a main blowing device (12) configured for blowing gas at least in a main blowing direction (121) in a detachment zone (C) through which the conveyor belt (11) travels, characterized in that the transfer surface is made of a mesh material, in that the gas is blown by the main blowing device (12) through the transfer surface at least from the second side to the first side of the transfer surface.

## Description

### FIELD OF THE INVENTION

The present invention relates to a device and a process for transferring insects from a container into another container.

More specifically, the invention relates to a device and a process for automatically transferring insects at a stage other than the adult stage from a container, for example an egg-growth chamber, into another container, such as a larval chamber.

### TECHNOLOGICAL BACKGROUND

The life cycle of an insect comprises three or four stages : the egg stage, the larval stage, the nymph stage in the case of Endopterygota, the adult or imaginal stage (adult insect and imago being synonymous). The environmental conditions required for an optimal development of the insects aren't the same at the three or four stages of the insect's life cycle. Furthermore, a population of insects is never completely synchronous : all the eggs don't hatch at the same time, all the adults don't emerge at the same time, etc.

Insect rearing requires as a consequence to transfer insects passing from one stage to another from a container to another container. In case the stage reached is the adult stage, the insects are very mobile so that they can spontaneously move from one container to another one, for example by flying.

At the other stages, insects - and in particular Hermetica Illucens - are either not very mobile, or almost or totally immobile. For example, Hermetica Illucens larvae can crawl but aren't prone to spontaneously move from one location to another. As a consequence, the insects must be transferred by adapted means from a container to another one when they reach a new stage of their life cycle except the adult stage.

Different devices and processes are known for automatically transferring insects from a container to another container.

WO2019/008591 relates to a device and a method for sorting insects, such as mosquitoes, with a view to quality control of production. The insects are sorted either at the emergence stage or at another stage (hatching, larval stage) after cooling so that they are relatively immobile. The insects are sorted according to their sex on the basis of an image analysis device. The insects to be sorted leave their container through a hatch and land on a conveyor which carries them to an imaging device. A flow of cold air can be used to knock the insects onto the conveyor, and optionally the container can be vibrated / shaken.

This method is not necessarily very suitable for insect larvae, which are relatively fragile. For example, the flow of cold air can dehydrate the larvae and the vibrations can disrupt their development, resulting in a relatively high loss rate.

WO2019/154563 discloses a device for rearing larvae, for example black soldier fly larvae, as well as the corresponding process.

The process comprises:
- Inducing oviposition in suitable devices;
- Let the eggs hatch and the larvae fall by gravity onto a conveyor belt;
- Transport the larvae to a container placed at one end of the conveyor belt;
- Count the larvae before collecting them in a new container, until the desired filling level is reached.

To solve the problem of larvae sticking to the conveyor belt, the inventors of WO2019/154563 tested different methods: a) blowing a concentrated air column on the conveyor belt ; b) Place a scraper near the conveyor belt; 3) Vibrate the conveyor belt at the tipping point; the third method having been deemed the only satisfactory one.

The three methods present the same drawbacks as for WO2019/008591.

The invention aims at proposing a device and a method for transferring insects at a stage other than the adult stage that reduces the damages caused to the insects relatively to prior art devices and methods, and/or ensuring that the transfer is more effective and/or that the number and/or the type of insects transferred is better controlled than in prior art.

### SUMMARY OF THE INVENTION

Hence, the invention relates to a device for transferring insects from an initial container into a final container comprising: - a conveyor belt (11) comprising a transfer surface having a first and a second opposite side, the first side being configured for receiving insects from the initial container, the insects being received at an entry area (A) of the first side of the conveyor belt (11), the first side of the conveyor belt being further configured for transporting the insects from the entry area (A) to an exit area (B) where the insects are configured to be received in the final container, and
a main blowing device (12) configured for blowing gas at least in a main blowing direction (121) in a detachment zone (C) through which the conveyor belt (11) travels, characterized in that the transfer surface is made of a mesh material, in that the gas is blown by the main blowing device through the transfer surface at least from the second side to the first side of the transfer surface. Blowing gas (for example air) on the insects through the transfer surface, which is made possible thank to the mesh material, allows to unstick them from the transfer surface with a more moderate and smoother air flow than if the air was blown laterally or equivalently in a direction tangent to the transfer surface like with conveyor belts made of plain materials. The gas flow thus doesn't decrease (or decreases very little) the insect quality and/or survival rate while ensuring a very high transfer rate.

Moreover, the parameters of the gas blown on the insects can be controlled so as to create optimal environmental conditions for preserving the insects to be transferred (such as temperature, relative humidity, etc).

In a particular embodiment of the device for transferring insects, the main blowing direction is locally orthogonal to the transfer surface along at least part of the detachment zone. This embodiment allows to control both the power consumption required to obtain an efficient air flow and to control the pressure of the air flow felt by the insects, an air flow at orthogonal or at an angle with the direction normal to the transfer surface being optimal depending among others on the insects.

In a particular embodiment of the device for transferring insects, the transfer surface is made of meshed stainless steel. Stainless steel is corrosion resistant and easy to wash. It is also rigid enough to form a rather flat transfer surface, with almost no hollows and bumps which would decrease the precision of the transfer.

In a particular embodiment, the device for transferring insects comprises a pulsed gas ramp.

A pulsed gas flow requires less power consumption than a continuous one and helps limit the insects dehydration associated with the gas blowing.

In a particular embodiment of the device for transferring, the main blowing device blows gas from both sides of the transfer surface. Blowing gas in two opposite senses can help control the gas flux of the main blowing device and/or maintain the insects on the transfer surface despite their unsticking and thereby increase the transfer precision.

In an embodiment, the device for transferring insects further comprises a cleaning zone comprising a cleaning device, said cleaning device being configured to clean the conveyor belt while said conveyor belt travels.

In an embodiment, the cleaning device is a steam supply device configured to inject steam on the conveyor belt as said conveyor belt travels.

In an embodiment, the device for transferring insects further comprises a drying zone (F), said drying zone comprising a drying device configured to dry the conveyor belt after said conveyor belt has passed through the cleaning zone.

In a particular embodiment, the device for transferring insects further comprises counting means in a counting zone through which the conveyor belt travels, located at least partially before the detachment zone and configured for counting the insects transferred into the final container. The detachment zone ensures that the insects counted in the counting zone are unstuck from the transfer surface in the detachment zone. As a consequence, the insects transfer rate is correlated with the number of insects counted. This means that the counting means and the detachment zone cooperate to increase the precision of the transferring of the insects compared to prior art.

In a particular embodiment of the device for transferring insects, the counting means comprise an image acquisition device. This makes it possible to automatically count the insects in the counting zone.

In a particular embodiment, the device for transferring insects further comprises an additional blowing device blowing gas in a direction locally tangent to the transfer surface along at least part of the counting zone.

The gas blown by such an additional blowing device can be blown with a low pressure and a low flow velocity, these parameters being however high enough to stimulate the insects. This stimulation facilitates the ungrouping of the insects and their counting.

In a particular embodiment of the device for transferring insects according to any of the preceding embodiments, the insects are chosen in the list: Hermetica Illucens, musca domestica and fruit fly, at a stage other than the adult stage and in which the gas flow of the main blowing device (12) has a pressure lower than 6 bars and/or a velocity lower than 5 meters per second.

These insects at theses stages are known to produce much glue, rendering their unsticking from the transfer surface particularly useful. The pressure and velocity ranges have been observed to achieve efficient unsticking with high survival rates.

Last, the invention relates to a device for rearing insects comprising at least one initial container, at least one final container and at least one device for transferring insects from at least one initial container into at least one final container according to any of the preceding embodiments.

This makes it possible to rear insects at different stages in different containers and different area, each area and/or each container being for example dedicated to a particular stage or rearing operation and transfer the insects from one container to another one with as few losses and as little impact as possible on the quality of the insects.

### BRIEF DESCRIPTION OF THE DRAWINGS

Embodiments of the invention will be described below, in relation to the following drawings :
Figure 1 is a side view of a first embodiment of the insects transfer device.
Figure 2 shows a side view of a second embodiment of the insects transfer device.
Figure 3a is a front view of an embodiment of the detachment zone C comprising a ramp of nozzles.
Figure 3b is the side view of an embodiment of the detachment zone C corresponding to the embodiment of Figure 3a.

On the drawings, the same reference signs show the same or similar objects.

### DETAILED DESCRIPTION

The invention deals with the optimization of insects rearing.

In general, the device and the process according to the invention are adapted for Dipteran insects at the larval or the nymph stage, such as the Black Soldier Fly (Hermetica Illucens), but other insects and/or other stages could be considered.

To facilitate understanding, the invention will often be described in a detailed manner in the case of Hermetica Illucens, but the person skilled in the art will easily generalize this description to other insects.

The life cycle of an insect comprises three or four stages : the egg stage, the larval stage, the nymph stage in the case of Endopterygota, the adult or imaginal stage (adult insect and imago being synonymous).

As described above, at the stages other than the adult stage, insects - and in particular Hermetica Illucens - are either not very mobile, or almost or totally immobile. For example, larvae can crawl but aren't prone to spontaneously move from one location to another. As a consequence, the insects must be transferred by adapted means form a container to another one when they reach a new stage of their life cycle except the adult stage.

Generally speaking, in a view of optimization of an insect rearing process requiring insect transfers, an insect transfer operation must be designed to minimize damages caused to the insects and simultaneously, to ensure that the transfer is effective and as far as possible, that the number and/or the type of insects transferred is controlled.

Numerous insect species produce one or more adhesive substance at one or more of the stages other than the adult stage, for example to secure their eggs or their cocoons. Several of these adhesive substances are described in Flora Borne et al. ; "The glue produced by Drosophila melanogaster for pupa adhesion is universal". Journal of Experimental Biology 15 April 2020; 223 (8): jeb220608. doi: https://doi.org/10.1242/jeb.220608.

As a consequence, the insects are relatively sticky at these stages and tend to adhere to the support or the container on or in which they are placed. This sticky (or slimy) character is an obstacle to the effective transfer of the insects from a container, called initial container, to another container called final container.

Vibrating the container or blowing air on the container containing the insects helps detach the insects from the initial container in order to transfer them. However, these methods are relatively traumatic for the insects and lead to too high loss rates and/or to insects of poor quality.

In order to reduce the impact of the transfer operation, the inventors have developed an insect transfer device 1 which makes it possible to reduce the air flow imposed on the insects and, in the cases where vibrations are required, to reduce the amplitude of the vibrations, reducing by the way the stress caused to the insects, in particular to the larvae.

The insect transfer device 1 according to the invention comprises a mesh conveyor belt 11.

The mesh conveyor belt 11 has two sides : a first side configured for receiving insects from the initial container and an opposite second side.

The mesh conveyor belt 11 forms a mesh transfer surface as it travels between an entry area A and an exit area B.

The mesh belt conveyor 11 receives larvae at the entry area B. The larvae are exiting the mesh belt conveyor 11 at the exit area B.

The mesh conveyor belt 11 circulates at a controlled speed. One or more motors may be used to make the mesh let conveyor 11 circulate.

The direction of circulation of the mesh conveyor belt 11 may always be the same. As an example, the mesh conveyor belt may circulate continuously such that a surface of the mesh conveyor belt 11 may begin to circulate from the entry area A toward the exit area B and continue circulate until it returns to the entry area A. Hence, the first side of the mesh conveyor belt receives larvae while it circulates from entry area A to exit area B, where the larvae exit the mesh conveyor belt. The mesh conveyor belt circulating from exit area B to entry area A may then be free from larvae.

The insects to be transferred pass from the initial container onto the first side of the mesh conveyor belt 11 in the entry area A. In a particular embodiment, the content of the initial container is automatically dumped onto the entry area A of the conveyor belt 11.

In another embodiment, the initial container is placed above the entry area A so that the insects can spontaneously fall onto the entry area A.

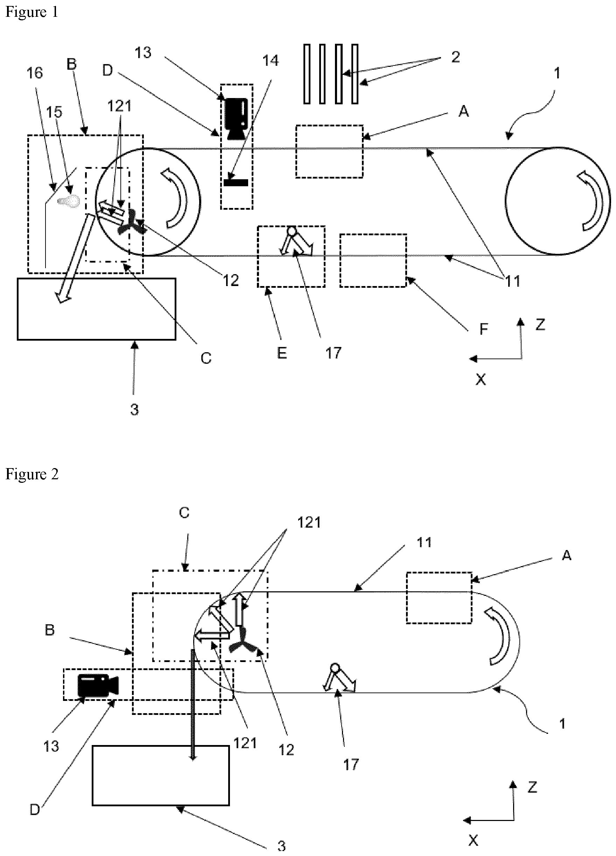

For example, the initial container can comprise one or more oviposition devices 2, as represented on figure 1, that are arranged above the entry area A so as to permit the larvae emerging from the eggs to fall on the entry area A due to their limited movements and to gravity.

The mesh conveyor belt 11 comprises a mesh material. The mesh material can be any suitable material such as a plastic material or a textile material or a metallic material.

The mesh can be made of a non adhesive material. For example, the mesh can be coated with polytetrafluoroethylene (PTFE).

In a particular embodiment, the mesh conveyor belt 11 may comprise stainless steel. This not only facilitates maintenance, but also provides sufficient rigidity to limit the undulations of the transfer surface formed by the conveyor belt 11, and as a consequence allows better control of the transfer. Indeed, if the transfer surface forms hollows and bumps, the hollows can correspond to unwanted insect accumulation areas, for which the precision of the insect transfer process cannot be as high as for other zones, all things equal otherwise.

Stainless steel is also corrosion resistant, making this material suitable for insects requiring high humidity levels and/or insect rearing installations comprising areas where high levels of ammonia are emitted and can spread up to the mesh conveyor belt 11.

The characteristic dimensions of the lattice of the mesh material of the mesh conveyor belt 11 are smaller than at least one of the characteristic dimensions of the insects to be transferred, so that the insects cannot fall through the pores of the mesh once on the mesh conveyor belt 11.

The conveyor belt 11 travels through a detachment zone C between the entry area A and the exit area B. The insect transfer device 1 comprises a main air blowing device 12 configured for blowing air in the detachment zone C at least in a so-called main blowing direction 121, through the mesh at least from the side of mesh belt conveyor 11 that is opposite to the side on which the insects are arranged, that is : through the mesh from the second side to the first side.

This is made possible due to the choice of a mesh material for the conveyor belt 11.

In a particular embodiment, the main blowing direction 121 is locally substantially orthogonal to the transfer surface in at least part of the detachment zone C. In particular, this means that the main blowing direction 121 forms with the direction (NN') locally orthogonal to the transfer surface an angle β that is lower than 45°, lower than 40°, lower than 35°, lower than 30°, lower than 25°, lower than 20°, lower than 15°, lower than 10°, lower than 5°, or equal to 0°.

The air may be blown by means of suitably arranged point nozzles, flat air blade nozzles or equivalents, providing uniform air coverage of the transfer surface and eliminating the possibility for zones with reduced air exposure.

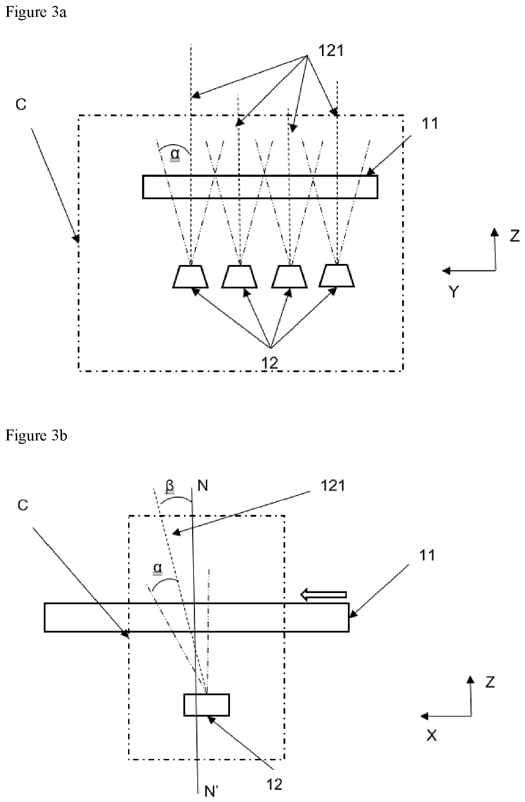

In a particular embodiment, as represented on figures 3a and 3b, the air can be blown from a point nozzle into an emission cone having :
- an emission axis, corresponding to the main blowing direction 121, locally substantially orthogonal to the transfer surface. That is to say, the emission axis can be locally exactly orthogonal to the transfer surface or form an angle β less than 45° with the direction (NN') locally normal to the transfer surface
- and a cone opening angle α of at least 0°, or at least or less than 5°, at least or less than 10°, at least or less than 15°, at least or less than 20°, at least or less than 25° and up to or less than 30°. Figure 3b represents a case where the transfer surface is substantially plane (or flat) all along the detachment zone C. Of course, depending on the composition of the transfer surface and on its tension, the transfer surface can be only approximately plane and deform more or less, in particular under its own weight.

In a particular embodiment, if the transfer surface is not comprised in a plane along the detachment zone C, the main air blowing direction 121 can evolve along the detachment zone C so as to be always locally substantially orthogonal to the transfer surface, as shown on Figure 2. A main blowing direction 121 at least locally orthogonal to the transfer surface is allowed by the mesh conveyor belt 11. Air can indeed circulate from one side of the transfer surface to the other through the pores of the mesh material, which is not the case with a continuous solid material.

Compared to prior art in which the main blowing direction 121 is tangent to the transfer surface, the velocity of the air flow and/or the air flow rate can be reduced, causing less damages to the insects, such as dehydration for example.

As a consequence, the insect transfer rate and/or the quality of the insect transferred is improved compared to prior art devices.

By way of example, the pressure of the air of the main blowing device 12 (measured right at the exit point of the main blowing device 12) is less than 6 bars, less than 5 bars, less than 4 bars, less than 3 bars, less than 2 bars.

By way of example, the velocity of the air of the main blowing device 12 (measured right at the exit point of the main blowing device 12) is less than 5 meters per second, less than 4 meters per second, less than 3meters per second, less than 3 meters per second, less than 1 meters per second. In a particular embodiment, the main air blowing device 12 is a pulsed air ramp that creates a shock on the insects strong enough for unsticking them. In this case, the insects are very briefly exposed to the main air flow. In other words, the fraction of the transfer surface exposed to the air flow created by the main air blowing device 12 can be very limited in case of an air ramp. The dehydration of the insects is consequently very limited, permitting high survival rates of the insects after transfer and a better growth of the transferred insects compared to prior art.

As an additional benefit, using pulsed air instead of continuous air flow reduces the overall mass of air needed and the power consumption. Hence, the capacity and operational costs of the air blowing device required could be proportionally reduced.

In a particular embodiment, the detachment zone C is close to the exit area B.

The detachment zone C can overlap at least partially with the exit area B as shown on Figure 2. The detachment zone C can also be entirely enclosed in the exit area B as shown on Figure 1.

In a particular embodiment, at least one parameter of the list : temperature, moisture, pressure, chemical composition, characterizing the air flow produced by the main air blowing device 12 is fixed and controlled with an air parameters control device. These parameters can depend on the insect and on the insect's life stage at which the transfer occurs.

For example, in the case of Hermetia Illucens neonates, the temperature of the air flow can be chosen in the range [28°C ; 30°C] and the relative humidity of the air flow can be in the range [0%, 80%]. In a particular embodiment, the air blown by the main blowing device 12 may be relatively dry, which means that the relative humidity can be lower than 40%, lower then 35 %, lower than 30%, lower than 25%, lower than 20 %, lower than 15%, lower than 10%.

In particular embodiment, the ratio pores surface / total surface of the conveyor belt 11 can be chosen in the range [30%, 50%] or even in the range [35%; 40%]. The inventors have observed that this range makes it possible to have, in combination with the main blowing device 12, an air flow smooth enough for preserving the insects and efficient enough to unstick them.

More specifically, in the case of Hermetica Illucens larvae, a mesh grid (or equivalently a mesh lattice):
- based on a square motif of 0,1mm*0,1mm,
- and where 38% of this square motif is a clear opening,
is adapted for both retaining the larvae and establish an optimal airflow to unstick them.

A light source 15 may be placed in exit area B, optionally in the detachment area C, in order to illuminate the transfer surface, as shown on Figure 1. In this case, as the neonates are photophobic, they start instinctively wiggling and moving in order to try to escape from the light. This wiggling additionally helps unstick them from the transfer surface and allows to reduce the pressure and/or the velocity of the air blown by the main air blowing device 12.

In a particular embodiment, the light spectrum of the light source 15 contains UV wavelengths. In a particular embodiment, the light source 15 illumination occurs on the transfer surface before the blowing of the main air blowing device 12.

In a particular embodiment, the insect transfer device comprises an additional blowing device configured to blow air on the insects in a so-called additional direction opposite to the main blowing direction in the detachment area C. The additional blowing device cooperates with the main air blowing device 12 to help maintain the insects above the transfer surface while they are unstuck from it. In this case, air is blown from both sides of the transfer surface.

Once unstuck, the insects can be transferred easily into a final container since they do not adhere to the transfer surface. In order to facilitate this, the air of the main blowing device can be directed in such a way that it enters the final container with airflow speed low enough to allow for efficient collection of the insects in the container.

For example, in case of Black Soldier Fly larvae right after the eggs have hatched, a larvae rearing box 3 can be arranged under the end of the mesh conveyor belt 11 proximal to the exit area B, as represented on Figure 1 and Figure 2 : the larvae are unstuck when reaching the detachment zone C, and they can afterwards in exit area B fall under the influence of gravity into the larvae rearing box 3.

In a particular embodiment, the insect transfer device 1 can further comprise counting means 13 in order to control the population filling a final container.

The counting means 13 can be placed in a counting zone D located at least partially after the detachment zone C.

A case where the counting zone D is before detachment zone C is represented on Figure 1 whereas on Fig. 2 the entire counting zone D is located after the detachment zone C.

The counting zone D can be located at least partially before the exit area B as shown on Figure 1. The counting zone D can be overlapping at least partially with the exit area B as shown on Figure 2.

On one hand, in case the detachment zone C is at least partially located after the counting zone D, the unsticking of the insects in the detachment zone C helps ensuring that all or almost all the insects that have been counted in the counting zone D are transferred into the final container.

On the other hand, placing the counting means 13 at least partially after the detachment zone C can also further improve the accuracy of the counting since the unsticking of the insects help the insects ungroup, in particular in the case of larvae that are prone to aggregation. As a consequence, insects that were previously aggregated and would have been counted as an only insect will in this case be counted correctly. This is to say : whatever the position of the counting means 13 (at least partially before or at least partially after the detachment zone D), the main air blowing device 12 of the detachment zone D and the counting means 13 cooperate to improve the accuracy of the insects transfer.

The counting means are configured to count the insects that are going to be effectively transferred into the final container either while they are still on the transfer surface or while they are falling from the transfer surface into the final container.

The counting means 13 can comprise optical means, such as a camera, configured to acquire one or more images to be analyzed for counting the insects.

In another embodiment, the additional blowing device can be configured to blow air on the insects in at least one lateral direction or in other words in a direction parallel to the transfer surface. The additional blowing device can be placed right before the counting zone D. This allows to stimulate the larvae before counting them. The increased movement of the larvae facilitates the subsequent counting.

The flow velocity of the air blown by the additional blowing device can in this case can be as low as 1 meter per second (1 m/s) or still less since this additional blowing device is not intended for unsticking the insects.

Deflectors 16 can be fitted to exit area B to direct the larvae into the final container and eliminate any chance of larvae being blown away and falling out of it. The deflectors cooperate with the main air blowing device 12, the air flow of which they quench and direct (and hence also the unstuck insects) into the final container. The deflectors may be designed with regard to the main blowing direction 121 and/or to the emission volume of the air flow of the main air blowing device 12.

In an embodiment, a wall may be provided at the level of the exit area B, where the mesh conveyor belt 11 turns. The wall may be provided to close a gap between the exit area B and the final container to ensure that the larvae exiting the exit area B fall in the final container. Advantageously, the wall is made in a material having a very low coefficient of friction, to avoid the larvae to stick to said wall. The material may be Teflon. The wall may be inclined to provide a slippery slope for the larvae between the exit area B and the final container.

The mesh conveyor belt 11 can be cleaned in a cleaning zone E to make sure that no remaining larvae (dead or alive), frass and/or other residues are stuck on the belt. To this aim, the device comprises steam supply device 17 configured to supply steam to the mesh conveyor belt 11. Advantageously, the steam supply device 17 is positioned such that it is configured to clean the sides of the mesh conveyor circulating from exit area B to entry area A, i.e. the part of the belt conveyor that does not transport larvae anymore. However, during the functioning of the device, some dead larvae, residues and/or frass may still be stuck on the surface of the mesh conveyor belt. The steam supply device 17 aims to clean such dead larvae, residues and/or frass.

More precisely, the steam supply device may comprise at least one steam supply nozzle configured to direct steam jets (represented by the arrows on figures 1 and 2) toward the mesh belt conveyor 11. Advantageously, the steam jets are delivered by the steam supply device 17 under pression such that it can help to unstick larvae, dead larvae, frass and/or residues from the mesh belt conveyor.

The use of steam is particularly advantageous since steam can disintegrate the frass accumulated on the mesh belt conveyor. Advantageously, the steam supply device 17 may be turned on and off as needed.

After washing, the mesh conveyor belt 11 may be dried in a drying zone F. Typically, the drying zone can comprise a drying device configured to dry the mesh conveyor belt. For example, the drying device may be configured to supply warm air toward the mesh conveyor belt to dry it.

A plurality of steam supply devices 17 may be provided along the opposite side of the mesh conveyor belt, advantageously upstream of the drying zone F.

The use in conjunction of the steam supply device 17 and the drying zone F is that the mesh conveyor belt 11 may be cleaned while still receiving larvae from the initial container. Hence, there is no need to stop the convey of the larvae to clean the mesh conveyor belt, leading to a continuous supply of larvae in the final container.

The invention also deals with a device for rearing insects comprising at least one initial container, at least one final container and at least one device for transferring insects (1) from at least one of the initial containers to at least one of the final containers.

## Claims

1. Device for transferring insects from an initial container into a final container comprising:
- a conveyor belt (11) comprising a transfer surface having a first and a second opposite side, the first side being configured for receiving insects from the initial container, the insects being received at an entry area (A) of the first side of the conveyor belt (11), the first side of the conveyor belt being further configured for transporting the insects from the entry area (A) to an exit area (B) where the insects are configured to be received in the final container, and
- a main blowing device (12) configured for blowing gas at least in a main blowing direction (121) in a detachment zone (C) through which the conveyor belt (11) travels, **characterized in that** the transfer surface is made of a mesh material, **in that** the gas is blown by the main blowing device (12) through the transfer surface at least from the second side to the first side of the transfer surface.

2. Device for transferring insects according to claim 1 in which the main blowing direction (121) is locally orthogonal to the transfer surface along at least part of the detachment zone (C).

3. Device for transferring insects according to claim 1 or claim 2 in which the transfer surface is made of meshed stainless steel.

4. Device for transferring insects according to any of claims 1 to 3 in which the main blowing device (12) blows the gas in a pulsed manner.

5. Device for transferring insects according to any of claims 1 to 4 in which the main blowing device (12) blows gas from both sides of the transfer surface.

6. Device for transferring insects according to any of claims 1 to 5, further comprising a cleaning zone (E) comprising a cleaning device, said cleaning device being configured to clean the conveyor belt (11) while said conveyor belt travels.

7. Device for transferring insects according to claim 6, wherein the cleaning device is a steam supply device (17) configured to inject steam on the conveyor belt as said conveyor belt travels.

8. Device for transferring insects according to claim 6 or 7, further comprising a drying zone (F), said drying zone comprising a drying device configured to dry the conveyor belt after said conveyor belt has passed through the cleaning zone (E).

9. Device for transferring insects according to any of claims 1 to 8 further comprising counting means (13) in a counting zone (D) through which the conveyor belt (11) travels, located at least partially before the detachment zone (C) configured for counting the insects transferred into the final container.

10. Device for transferring insects according to claim 9 in which the counting means (13) comprise an image acquisition device.

11. Device for transferring insects according to any of claims 1 to 10 further comprising an additional blowing device blowing gas in a direction locally tangent to the transfer surface.

12. Device for transferring insects according to any of the preceding claims in which the insects are chosen in the list: Hermetica Illucens, musca domestica and fruit fly, at a stage other than the adult stage and in which the gas flow of the main blowing device (12) has a pressure lower than 6 bars and/or a velocity lower than 5 meters per second.

13. Device for rearing insects comprising at least one initial container, at least one final container and at least one device for transferring insects (1) from at least one initial container to at least one final container according to any of claims 1 to 12.
